# EUROPEAN PATENT SPECIFICATION

(11) **EP 0 687 732 B1**
(45) Date of publication and mention of the grant of the patent: **06.05.1998**
(21) Application number: 95303772.8
(22) Date of filing: 02.06.1995
(51) Int. Cl.: C12N 15/56, C12N 9/38

(54) **Hyperthermostable beta-galactosidase gene**
Für ein hyperthermostabiles Beta-galaktosidase kodierendes Gen
Gène codant pour une bêta-galactosidase hyperthermostable

(30) Priority: 14.06.1994 JP 154356/94
(43) Date of publication of application: 20.12.1995
(73) Proprietor: TAKARA SHUZO CO. LTD., Fushimi-ku, Kyoto-shi, Kyoto-fu (JP)
(72) Inventor: Shimada, Atsushi, Otsu-shi, Shiga-ken (JP); Koyama, Nobuto, Uji-shi, Kyoto-fu (JP); Asada, Kiyozo, Koga-gun, Shiga-ken (JP); Odate, Miki, Kusatsu-shi, Shiga-ken (JP); Hashino, Kimikazu, Takatsuki-shi, Osaka-fu (JP); Kato, Ikunoshin, Uji-shi, Kyoto-fu (JP)
(74) Representative: Wakerley, Helen Rachael

(56) References cited:
- EP-A- 0 592 158
- EP-A- 0 606 008
- FEMS MICROBIOLOGY LETTERS, vol. 109, 1993 pages 131-138, JOSEF GABELSBERGER ET AL. 'Cloning and characterization of beta-galactoside and beta-glucoside hydrolysing enzymes of Thermotoga maritima '
- JOURNAL OF APPLIED BIOCHEMISTRY, vol. 2, no. 5, 1980 pages 390-397, VINCENZO BUONOCORE ET AL. 'A constitutive Beta-galactosidase from the extreme thermoacidophile archaebacterium Caldariella acidophila: Properties of the enzyme in the free state and in immobilized whole cells'
- INTERNATIONAL JOURNAL OF BIOCHEMISTRY , vol. 5, 1974 pages 629-632, ROBERT P. ERICKSON 'Stability of Escherichia coli Beta-galactosidase in Sodium dodecyl sulfate'

## Description

### Field of Industrial Application

The present invention relates to a gene encoding an SDS-resistant hyperthermostable β-galactosidase, a method of cloning the galactosidase gene with the use of the gene or a part thereof and a genetic engineering process for producing the enzyme which is useful in the fields of, for example, food industry and sugar engineering.

### Prior Art Technology

β-Galactosidase, which is an enzyme capable of decomposing β-galactoside, has been found out in animals, plants and microorganisms. It is known that this enzyme occurs particularly in bacteria such as Escherichia coli, Streptococcus lactis, Bacillus subtilis, Streptococcus thermophilus and Sulfolobus solfataricus. This β-galactosidase is applied to the production of low-lactose milk by taking advantage of its ability to hydrolyze lactose into galactose and glucose. It is also applied to the production of galactose or glucose from lactose contained in milk serum which is formed in a large amount in the process of producing cheese.

To apply β-galactosidase to food processing, therefore, it has been demanded to develop an enzyme which can withstand the use at a high temperature from the viewpoint of preventing contamination with microorganisms during the processing and another viewpoint of elevating the solubility of lactose which serves as a substrate.
Further, in recent years, various sugar compound productions are conducted with the use of a β-galactosidase glycosyltransfer reaction (Japanese Patent Laid-Open No. 25275/1994 and Japanese Patent Laid-Open 14774/1994). Thus, the development of highly thermostable enzymes is desired.
For example, a β-galactosidase originating in Sulfolobus solfataricus [European Journal of Biochemistry, 187, 321 - 328 (1990)] is a thermophilic enzyme having an activity at a temperature of 90 °C. However, its activity falls to about 50% after treating at 85 °C for 180 minutes. FEMS Microbiology Letters 1993, Vol. 109, pages 131-138 describes the cloning and characterisation of β-galactoside and the isolation of genes encoding β-galactosidase activity from a gene library of Thermotoga maritima.
it is described in, for example, European Journal of Biochemistry, 213, 305-312 (1993) that β-galactosidase derived from the hyperthermophilic bacterium Pyrococcus furiosus exhibits its activity at high temperatures, thereby ensuring a high thermostability. The inventors discovered a hyperthermostable β-galactosidase having a residual activity ratio of about 80% even after treatment at 90 °C for 120 minutes and succeeded in isolating three types of β-galactosidases (European Patent Laid-Open No. 0592158A2).

These three types of β-galactosidases are all hyperthermostable, and one of them is a β-galactosidase has an extremely high stability and exhibits its activity even in the presence of 1% Sodium dodecyl sulfate (SDS).

### Problem to be Solved by the Invention

As mentioned above, a thermophilic and thermostable enzyme is demanded in the food processing and sugar compound production conducted at high temperatures. Further, if an enzyme holds its activity even in the presence of SDS known as a powerful surfactant, its application range can be widened.

It is desired to isolate a gene encoding a β-galactosidase having an improved thermophilicity, an excellent thermostability and the resistance to surfactants and to an industrial process for producing a hyperthermostable β-galactosidase with the use of the above gene.

### Means for Solving the Problem

The first aspect of the present invention provides a DNA comprising:
(a) the DNA sequence of SEQ ID NO 2, or a fragment thereof;
(b) a DNA Sequence encoding the amino acid sequence of SEQ ID NO 1 or a fragment thereof;
(c) a DNA sequence encoding an amino acid sequence resulting from deletion, addition, insertion or substitution of one or more amino acids in the amino acid sequence of SEQ ID NO 1; or
   a DNA sequence capable of hybridizing of any one of (a) to (c),
   the DNA having a sequence encoding a polypeptide possessing SDS-resistant hyperthermostable β-galactosidase activity of about 90% after having been treated in the presence of 1% SDS at 90°C for 120 minutes.
The present invention further provides a method of cloning a DNA having a sequence encoding a polypeptide possessing SDS-resistant hyperthermostable β-galactosidase activity in the presence of 1% SDS at 90°C for 120 minutes, which comprises using a DNA according to the first aspect of the invention or a part thereof as a probe or primer.
The present invention still further provides a process for producing a polypeptide possessing SDS-resistant hyperthermostable β-galactosidase activity in the presence of 1% SDS at 90°C for 120 minutes, which comprises culturing a transformant, into which a recombinant plasmid containing a DNA according to the first aspect of the invention has been introduced, and harvesting an SDS-resistant hyperthermostable β-galactosidase from the culture.

The hyperthermostable β-galactosidase gene which includes an isolated DNA encoding a hyperthermophilic β-galactosidase in this invention can be screened and obtained by the expression cloning method using cosmid vectors. Expression cloning is a method which can be used for cloning of the gene coding some enzymes without any information about the primary structure of the target enzyme. For example, a pullulanase gene of Pyrococcus woesei (WO 92/02614) is cloned using the expression cloning method. However, the method cannot be applied to cloning of any type of enzyme because in case the plasmid vector is used for the method, a very suitable restriction enzyme is needed; It must cleave the target gene into small size enough to be inserted in a plasmid vector and neither cleave the target gene at inside. Furthermore, the method is complicated because it needs a number of clones.

Subsequently, the present inventors have attempted to isolate the β-galactosidase gene by screening β-galactosidase activities in a cosmid library constructed with Pyrococcus furiosus genomic DNA and the cosmid vectors in which larger DNA fragments (35 - 50 kbp) can be inserted than in plasmid vectors. By using cosmid vectors, dangers for cleaving the target gene encoding the enzyme by a restriction enzyme at inside decrease and the numbers of clones necessary to test can be reduced. On the contrary, dangers not to detect the enzyme activity because of low expression of the enzyme because the cosmid vectors has less copy numbers in host organisms than the plasmid vectors.

The present inventors sited in extreme high thermostability of the target enzyme and combined a process of cultivating the transformants in the cosmid library individually with a process of preparing the lysates which contain only the thermostable proteins. The group of these lysates is named as "cosmid protein library". By using the library for detection of the enzyme activity, detection sensitivity increases more than using colonies of the transformants and bad influences such as background by proteins from hosts or inhibition of enzyme activity can be deleted.

The inventors searched the cosmid protein library derived from Pyrococcus furiosus, and obtained one cosmid clone exhibiting a β-galactosidase activity, though weak, in the presence of 1% SDS.

Furthermore, the present inventors isolated the gene coding a hyperthermostable β-galactosidase from the DNA fragments inserted in the clones isolated above by making full use of various genetic engineering techniques, and determined the DNA sequence of the gene. And more, the present inventors succeeded in the expression of the hyperthermostable β-galactosidase with the use of the gene, thus completing the present invention.

By the way, the expression cloning method using cosmid vectors which is described here cannot be always applied to any thermostable enzyme. The result is determined by the property of the target gene. For the example, the present inventors attempted to isolate the gene encoding a α-glucosidase of Pyrococcus furiosus [Journal of Bacteriology, 172, 3654 - 3660 (1990)], but they didn't reach to the isolation of the gene.

Now, the present invention will be described in greater detail.

The microorganism to be used in the present invention is not particularly restricted, so long as it can produce a hyperthermostable β-galactosidase gene. For example, strains belonging to the genus Pyrococcus, i.e., hyperthermostable bacteria, such as Pyrococcus furiosus DSM 3638 and Pyrococcus woesei DSM 3773 are usable therefor. These strains are both available from Deutsche Sammlung von Mikroorganismen und Zellkulturen GmbH.

For example, a cosmid library of Pyrococcus furiosus gene can be prepared in the following manner. First, the genome gene of Pyrococcus furiosus DSM 3638 is partially digested by using an appropriate restriction enzyme, for example, Sau 3AI (manufactured by Takara Shuzo Co., Ltd.). After fractionating according to the size of 35 to 50 kbp, each DNA fragment thus obtained is ligated with an appropriate cosmid vector, for example, Triple Helix Cosmid Vector (manufactured by Stratagene). The Pyrococcus furiosus genome DNA fragments are first packaged in λ-phage particles by the in vitro packaging method and then an appropriate Escherichia coli strain, for example, Escherichia coli DH5αMCR (manufactured by BRL) is transformed with the obtained phage solution to thereby give the aimed cosmid library. Then cosmid DNAs are prepared from several colonies of the transformant and the insertion of the genome DNA fragments of 35 to 50 kbp into the transformant is thus confirmed. In general, 300 to 700 colonies may be incubated.

After the completion of the incubation of each colony, the incubated cells are collected. The cells are processed to the cosmid protein libraries by treating at 100 °C for 10 minutes, sonicating, and treating at 100 °C for 10 minutes once more. Then the β-galactosidase activity in the lysates obtained is determined in the presence of 1% SDS, whereby colonies expressing a hyperthermostable β-galactosidase which remains stable after treatment above described can be screened. The β-galactosidase activity is determined by, for example, using o-nitrophenyl-β-D-galactopyranoside or lactose (all manufactured by Nacalai Tesque) as a substrate at a reaction temperature of, for example,
95 °C. Next, the fragment inserted into the cosmid DNA of transformant showing the activity is analyzed.

The fragments inserted into the cosmid DNA of one transformant having exhibited an activity among 500 transformants prepared by the inventors are cleaved with the use of various restriction enzymes, and the resultant fragment group is inserted into a suitable vector. For example, the cosmid DNA prepared from the above-mentioned cosmid clone is digested with Hind III (manufactured by Takara Shuzo Co., Ltd.), and the obtained DNA fragments are inserted into the Hind III site of the plasmid vector pUC18 (manufactured by Takara Shuzo Co., Ltd.). Thus, a recombinant plasmid can be obtained.

Subsequently, this recombinant plasmid is introduced into the Escherichia coli JM109 (manufactured by Takara Shuzo Co., Ltd.) to thereby obtain a transformant, which is cultured and harvested. The activity of the β-galactosidase, a protein expressed in the cells, is assayed. The assay is conducted with respect to the cells and lysate thereof having undergone heat treatment at 100 °C for 10 minutes twice by using o-nitrophenyl-β-D-galactopyranoside as a substrate in the presence of 1% SDS. The activity is assayed by conducting the reaction at 95 °C for 30 minutes.

The above transformant lysate has no activity recognized. Then, the activity search was conducted in the same manner with the use of each of the restriction enzymes Acc I, Bgl II, Eco RV, Pst I and Hinc II (all manufactured by Takara Shuzo Co., Ltd.), but no activity was found.

The same search was conducted with the use of a restriction enzyme capable of providing longer DNA fragments than with the use of the above restriction enzymes, for example, Cla I (manufactured by Takara Shuzo Co., Ltd.). However, deletion was found in the insert fragments at the stage of insertion into the plasmid, and no activity was recognized. Next, the search was conducted in the same manner with the use of Sma I (manufactured by Takara Shuzo Co., Ltd.). As a result, activity was recognized in a plasmid having a DNA fragment of about 4 kbp inserted therein. This plasmid was designated plasmid pTG2S-112 by the inventors. By transforming Escherichia coli JM109 by this plasmid, a transformant designated as Escherichia coli JM109/pTG2S-112 by the present inventors can be obtained. This transformant is incubated and, after the completion of the incubation, the cells are collected. The β-galactosidase expressed in these cells remains stable irrespective of the heat treating in the presence of 1% SDS at 100 °C for 1O minutes twice. Thus the target hyperthermostable β-galactosidase has been expressed therein.

Further, the plasmid pTG2S-112 is digested with various restriction enzymes, and the resultant fragments are inserted in suitable vectors. The resultant recombinant plasmids are introduced into the Escherichia coli JM109, and the obtained transformants are cultured and harvested. The activity of β-galactosidase, a protein expressed in the cells, is assayed. Thus, a plasmid expressing hyperthermostable β-galactosidase can be searched for.

For example, the plasmid pTG2S-112 is digested with the restriction enzymes Eco81I (manufactured by Takara Shuzo Co., Ltd.) and Sma I. The resultant Eco81I-Sma I DNA fragment of about 2.0 kbp is purified and inserted in pUC18 to thereby obtain a recombinant plasmid.

Alternatively, with the utilization of the multicloning site of the vector (pUC18) region of pTG2S-112, pTG2S-112 is digested with the restriction enzymes Eco81I and Kpn I (manufactured by Takara Shuzo Co., Ltd.). The resultant Eco81I-Kpn I DNA fragment of about 4.7 kbp is purified, blunt-ended and selfligated. Thus, a recombinant plasmid containing the above-mentioned Eco81I-Sma I DNA fragment of about 2.0 kbp can be obtained.

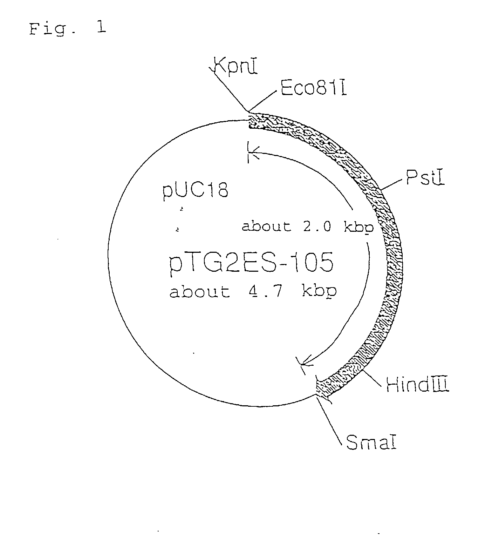

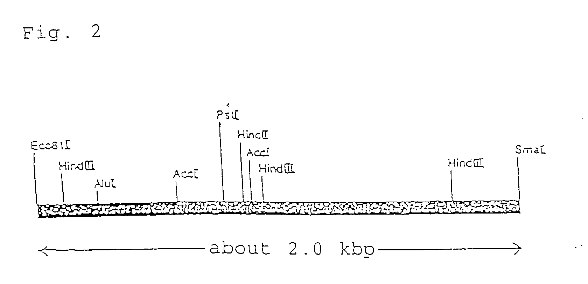

This plasmid is introduced into the Escherichia coli JM109, and the resultant colonies are assayed for the hyperthermostable β-galactosidase activities thereof. A plasmid is prepared from the colony having exhibited the activity. This plasmid is designated as plasmid pTG2ES-105. The Escherichia coli JM109 transformed with this plasmid is designated as Escherichia coli JM109/pTG2ES-105. This strain was deposited on April 20, 1994 at National Institute of Bioscience and Human-Technology Agency of Industrial Science and Technology (1-3, Higashi 1 Chome Tsukuba-shi Ibaraki-ken 305, JAPAN) under the accession number FERM BP-5023. A restriction enzyme cleavage map of the plasmid pTG2ES-105 is shown in Fig. 1, in which the thick solid line represents the fragment inserted in the plasmid pUC18.

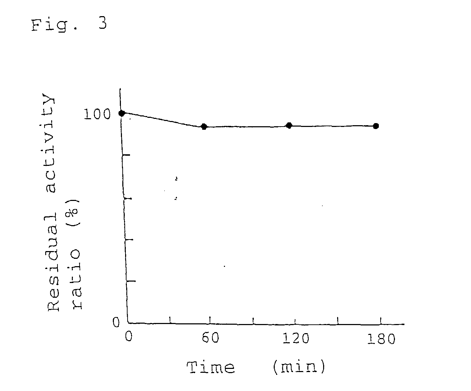

Fig. 2 shows a restriction enzyme cleavage map of the DNA fragment derived from Pyrococcus furiosus and inserted in the plasmid pTG2ES-105. That is, Fig. 2 is a view showing the restriction enzyme cleavage map of one form of the hyperthermostable β-galactosidase gene obtained according to the present invention. The β-galactosidase expressed in the cells obtained by culturing the transformant designated as Escherichia coli JM109/pTG2ES-105 followed by harvesting is stable irrespective of the heat treatment conducted in the presence of 1% SDS at 100 °C for 10 minutes twice. Thus the target hyperthermostable β-galactosidase has been expressed therein.

The hyperthermostable β-galactosidase is accumulated by culturing a transformant, into which a recombinant plasmid containing the hyperthermostable β-galactosidase gene has been introduced, e.g., Escherichia coli JM109/pTG2S-112 or Escherichia coli JM109/pTG2ES-105. The purification of the hyperthermostable β-galactosidase from the culture may be effected, for example, by disrupting the harvested cells by sonication, centrifuging the lysate and subjecting the resultant supernatant to gel filtration chromatography, ion exchange chromatography, hydrophobic chromatography or the like.

When the hyperthermostable β-galactosidase is to be purified in the present invention, in particular, it is advantageous to thermally treat the cells either before or after the ultrasonication, since the contaminating proteins are denatured thereby and thus the purification can be easily carried out.

The hyperthermostable β-galactosidase obtained by expressing a gene of the present invention, for example, a gene integrated in the plasmid pTG2ES-105 has the following physicochemical properties.

### (1) Action:

It has an action of hydrolyzing lactose into galactose and glucose. Further, it has an action of hydrolyzing o-nitrophenyl-β-D-galactopyranoside into o-nitrophenol and galactose.

Further, it has an action of hydrolyzing o-nitrophenyl-β-D-galactopyranoside into o-nitrophenol and galactose under 50 mM phosphate buffer (pH 7.0) containing 1% SDS.

### (2) Method for determining enzymatic activity:

### [(2)-a]

In the determination of enzymatic activity, the o-nitrophenyl-β-D-galactopyranoside hydrolyzing activity of an enzyme can be determined by spectroscopically monitoring o-nitrophenol formed via the hydrolysis. Namely, 5 µl of the enzyme solution of the present invention is added to 199 µl of a 100 mM phosphate buffer solution (pH 7.0) containing 112 mM 2-mercaptoethanol, 1 mM magnesium chloride and 1% SDS. Then 1 µl of a dimethyl sulfoxide solution containing 0.4 M o-nitrophenyl-β-D-galactopyranoside is added thereto. After effecting a reaction at 95 °C for 30 minutes, the reaction is ceased by adding 100 µl of 0.1 M sodium carbonate and the absorbance of the reaction mixture at 410 nm is measured to thereby determine the amount of the o-nitrophenol thus formed. One unit of the hyperthermostable β-galactosidase obtained in according to with the present invention is expressed in an amount of the enzyme whereby the absorbance at 410 nm can be increased by 1.0 at 95 °C within 1 minute. The enzyme obtained in the present invention has an activity of decomposing o-nitrophenyl-β-D-galactopyranoside at pH 7.0 at 95 °C in the presence of 1 % SDS.

### [(2)-b]

The o-nitrophenyl-β-D-galactopyranoside hydrolyzing activity of the β-galactosidase also can be determined by the method shown as follows; The enzyme reaction was started by adding 15 µl of a dimethyl sulfoxide solution containing 1 M o-nitrophenyl-β-D-galactopyranoside into 1485 µl of McIlvaine buffer solution (pH 5.0) containing the enzyme which is in a quartz cuvette for spectrometer to give the final concentration of o-nitrophenyl-β-D-galactopyranoside to 10 mM. Reaction was detected by monitoring change of absorbance at 410 nm versus time on spectrophotometer. Based on the change of absorbance at 410 nm per minute, o-nitrophenol released per minute was calculated by using absorbance coefficient determined previously. One unit of enzyme activity was defined as that amount required to catalyze the release of 1 µmol o-nitrophenol per minute.

The assay of enzymatic proteins was carried out by the use of a protein assay kit (manufactured by Bio-Rad Laboratories).

### (3) Thermostability:

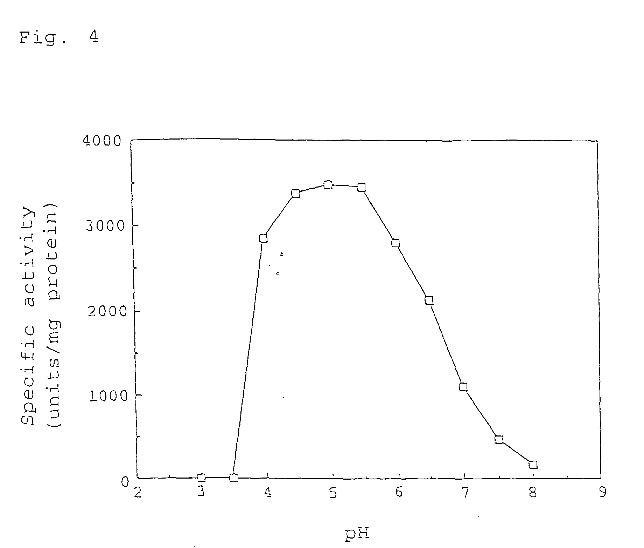

The thermostability was measured according to the following procedure in conformity with the method described in [(2)-b]. 1.5 ml of a McIlvaine buffer (pH 5.0) containing an enzyme is heated at 90 °C for a given period of time, and 1485 µl of the resultant solution is sampled therefrom. The sample is heated in a cuvette of a spectrophotometer at 90 °C for 5 minutes, and 15 µl of a dimethyl sulfoxide solution containing 1 M o-nitrophenyl-β-D-galactopyranoside is added thereto to initiate a reaction. This reaction may be traced by calculating a change is absorbance at 410 nm per minute and determining the amount of o-nitrophenol liberated per minute from a previously determined extinction coefficient of o-nitrophenol. The enzyme of the present invention has a residual activity ratio of about 100% even after heat treatment at 90 °C for 180 minutes as shown in Fig. 3. That is, Fig. 3 is a view showing the thermostability of the enzyme, in which the axis of ordinate indicates the residual activity ratio (%) and the axis of abscissa the period of time (min) for which the enzyme is treated at 90 °C.

### (4) Optimum pH ;

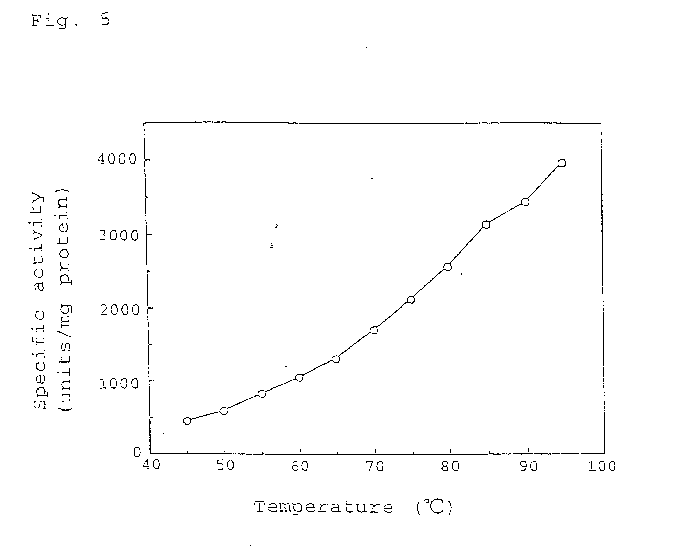

The optimum pH was measured in according to the method described in [(2)-b]. 2990 µl of McIlvaine buffer solution which was determined pH at appointed value (pH 4-8) and containing 10 mM o-nitrophenyl-β-D-galactopyranoside was incubated at 90 °C in the cuvette and the enzyme reaction was started by adding 10 µl of McIlvaine buffer solution (pH 5.0) containing the enzyme (150 units/ml) into the cuvette. Reaction was detected by monitoring change of absorbance at 410 nm versus time on spectrophotometer. Change of absorbance at 410 nm per minute was determined. Based on the change of absorbance at 410 nm per minute, o-nitrophenol released per minute was calculated by using absorbance coefficient determined at each pH condition. One unit of enzyme activity was defined as that amount required to catalyze the release of 1 µmol o-nitrophenol in a minute. As shown in Fig. 4 , the enzyme of the present invention shows its maximum activity at a pH range of from 4.5 to 5.5. Fig. 4 is a graph showing the optimum pH of an enzyme wherein the ordinate refers to the specific activity (units/mg protein), while the abscissa refers to treating pH.

### (5) Optimum temperature:

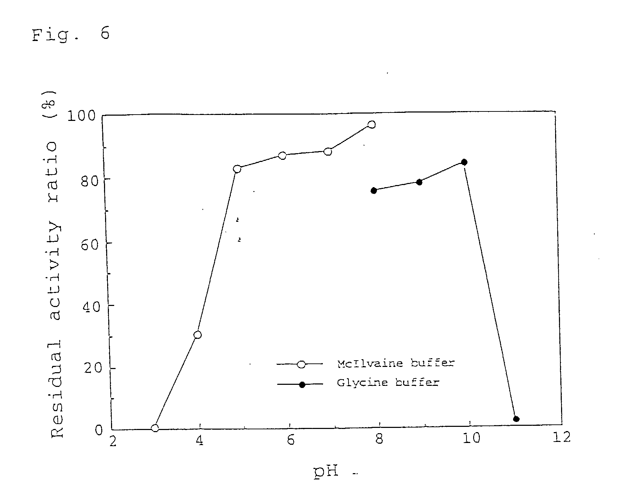

The optimum temperatures was measured in according to the described in [(2)-b]. 2990 µl of McIlvaine buffer solution (pH 5.0) containing 10 mM o-nitrophenyl-β-D-galactopyranoside was incubated at appointed temperature (45 °C - 90 °C) in the cuvette and the enzyme reaction was started by adding 10 µl of the enzyme (150 units/ml). Reaction was detected by monitoring change of absorbance at 410 nm versus time on spectrophotometer. Change of absorbance at 410 nm per minute was determined. Based on the change of absorbance at 410 nm per minute, o-nitrophenol released per minute was calculated by using absorbance coefficient determined at each temperature. One unit of enzyme activity was defined as that amount required to catalyze the release of 1 µmol o-nitrophenol per minute. As Fig. 5 shows, the enzyme of the present invention shows its maximum activity above 95 °C. Fig. 5 is a graph showing the optimum temperature of an enzyme wherein the ordinate refers to the specific activity (units/mg protein), while the abscissa refers to treating temperature (°C).

### (6) pH stability:

The pH stability was measured in according to the described in [(2)-b]. McIlvaine buffer solution (pH 3.0 - 8.0) containing 150 units/ml of the enzyme and glycine buffer solution (pH 8.0 - 11.0) containing 150 units/ml of the enzyme was incubated for 10 minutes at 90 °C. To start reaction, 10µl of the enzyme solution was added to 2990 µl of McIlvaine buffer solution (pH 5.0) which contained 10 mM of o-nitrophenyl-β-D-galactopyranoside and preincubated at 90 °C.

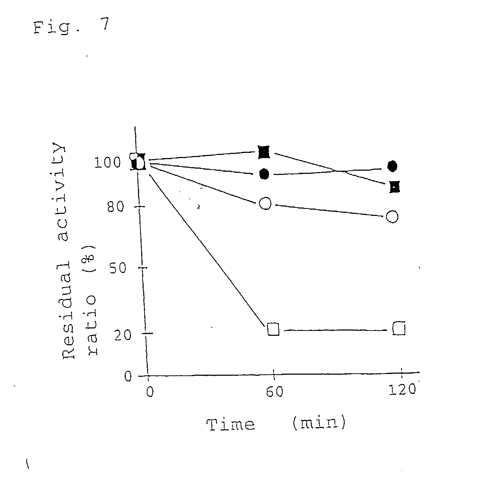

Reaction was detected by monitoring change of absorbance at 410 nm versus time on spectrophotometer. Change of absorbance at 410 nm in a minute was determined. Based on the change of absorbance at 410 nm per minute, o-nitrophenol released per minute was calculated by using absorbance coefficient determined previously. One unit of enzyme activity was defined as that amount required to catalyze the release of 1 µmol o-nitrophenol in a minute. As Fig. 6 shows, the enzyme of the present invention sustains its activity even after treating within a pH range of from 5.0 to 10.0 at 90 °C for 10 minutes. Fig. 6 is a graph showing the pH stability of the enzyme wherein the ordinate refers to the residual activity ratio(%), while the abscissa refers to treating pH.

### (7) Influence of various surfactants:

The thermostability of the enzyme in the presence of each of various surfactants was measured according to the following procedure in conformity with the method described in [(2)-b]. Sodium dodecyl sulfate (manufactured by Nacalai Tesque) was used as an anionic surfactant, hexadecyl trimethyl ammonium bromide (manufactured by Nacalai Tesque) as a cationic surfactant, polyoxyethylene (20) sorbitan monolaurate (manufactured by Wako Pure Chemical Industries, Ltd.) as a nonionic surfactant, and sodium cholate (manufactured by Nacalai Tesque) as a cholic surfactant.

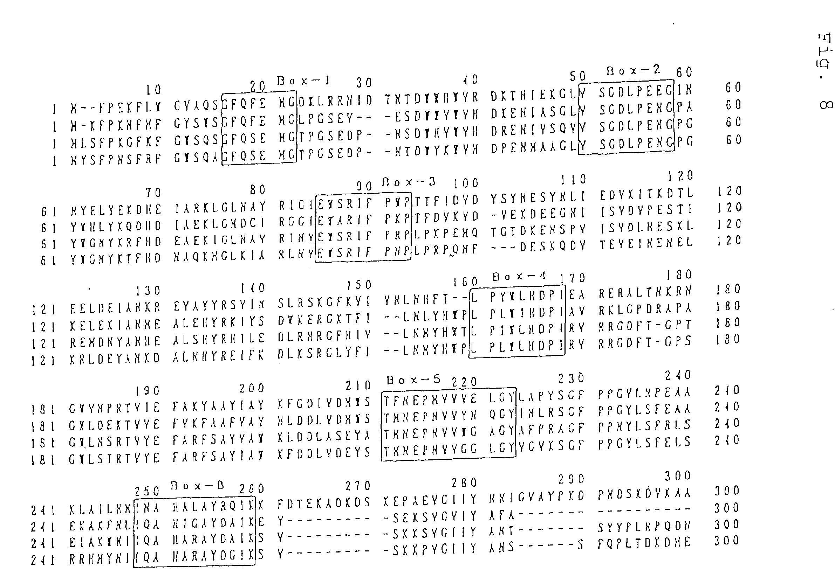

In the reaction solution, the concentration of the above surfactant was adjusted to 1%. 1.5 ml of a 50 mM phosphate buffer (pH 7.0) containing an enzyme is heated at 90 °C for a given period of time, and 1485 µl of the resultant solution is sampled therefrom. The sample is heated in a cuvette of a spectrophotometer at 90 °C for 5 minutes, and 15 µl of a dimethyl sulfoxide solution containing 1 M o-nitrophenyl-β-D-galactopyranoside is added thereto to thereby initiate a reaction. This reaction may be traced by calculating a change in absorbance at 410 nm per minute and determining the amount of o-nitrophenol liberated per minute from a previously determined extinction coefficient of o-nitrophenol. The enzyme of the present invention has a residual activity ratio of about 80% even after heat treatment at 90 °C for 120 minutes in the presence of any of the surfactants except hexadecyl trimethyl ammonium bromide, as shown in Fig. 7. In particular, the enzyme of the present invention has a residual activity ratio of about 90% even after heat treatment at 90 °C for 120 minutes in the presence of sodium dodecyl sulfate conventionally used for denaturation of proteins. Fig. 7 is a view showing the thermostability of the enzyme in the presence of each of the various surfactants, in which the axis of ordinate indicates the residual activity ratio (%) and the axis of abscissa the period of time (min) for which the enzyme is treated at 90 °C.

In the Fig. 7, the open square indicates hexadecyl trimethyl ammonium bromide, the solid square sodium dodecyl sulfate, the open circle polyoxyethylene (20) sorbitan monolaurate, and the solid circle sodium cholate.

### (8) Substrate specificity:

Substrate specificity is able to be determined by using p-nitrophenol-derivatives as shown in Table 1. The method is shown as follows;

1485 µl of 150 mM sodium citrate buffer solution (pH 5.0) containing the enzyme is added to a quartz cuvette for spectrometer. 15µl of 0.1M substrate solution shown in Table 1 is added to the enzyme solution and mixed. Immediately, reaction was detected by monitoring change of absorbance at 410 nm versus time on spectrophotometer. As a blank test, 1485 µl of 150 mM sodium citrate buffer (pH 5.0) not containing enzyme was used, and determination described above was performed. On the test, reaction was performed at 90 °C. One unit of enzyme activity was defined as that amount required to catalyze the release of 1 µmol p-nitrophenol per minute.

According to the method described above, Hydrolytic activity towards p-nitrophenyl-β-D-glucopyranoside (Glcp-βNp) , p-nitrophenyl-β-D-galactopyranoside (GalpβNp), p-nitrophenyl-β-D-mannopyranoside (ManpβNp), p-nitrophenyl-β-D-xylopyranoside (XylpβNp), p-nitrophenyl-β-D-fucopyranoside (FucpβNp), p-nitrophenyl-α-D-galactopyranoside (GalpαNp, all manufactured by Nacalai Tesque), was determined.

Results were shown in Table 1. The Table 1 shows specific activity [units/mg protein) towards above described substrates and relative activity (%).

**[Table 1]**

| Specific activity of the enzyme | | |
|---|---|---|
| Substrate | Specific activity (units/mg) | Relative activity (%) |
| GalpβNp | 192 | 100 |
| GlcpβNp | 512 | 267 |
| ManpβNp | 12.8 | 6.7 |
| XylpβNp | 51.2 | 26.7 |
| FucpβNp | 0 | 0 |
| GalpαNp | 0 | 0 |

Further, the enzymolytic activity of the enzyme was tested with the use of the following natural substrates. Specifically, each of lactose, cellobiose, methyl-β-D-glucose, salicin, arbutin, sucrose and maltose (all manufactured by Nacalai Tesque) as the substrate was dissolved in 1 ml of a 150 mM sodium citrate buffer (pH 5.0) in the final concentration of 50 mM. Each of carboxy methylcellulose (manufactured by Wako Pure Chemical Industries, Ltd.), Avicel (manufactured by Funakoshi Pharmaceutical Co., Ltd.) and laminarin (manufactured by Nacalai Tesque) was dissolved in the buffer in the final concentration of 17 g/l. Each of the above substrate solutions was heated to 90 °C, and 15 µl (about 45 mU) of a phosphate buffer (pH 7.0) of an enzyme was added thereto to effect a reaction at 90 °C for 30 minutes. The reaction was terminated by cooling with ice. The amount of glucose liberated in the reaction fluid was determined by the use of Glucose B Test Wako (manufactured by Wako Pure Chemical Industries, Ltd.). Table 2 shows the relative activities (%) determined with respect to the other substrates when the lactose hydrolyzing activity is taken as 100%.

**[Table 2]**

| Substrate specificity of the enzyme | |
|---|---|
| Substrate | Relative activity (%) |
| Lactose | 100 |
| Cellobiose | 136 |
| methyl-β-D-glucoside | 10.2 |
| Salicin | 69.6 |
| Arbutin | 6.1 |
| Sucrose | 1.9 |
| Maltose | 1.9 |
| Carboxymethyl-cellulose | 0 |
| Avicel | 0 |
| Laminarin | 1.3 |

### (9) Characteristics of amino acid sequence:

With respect to the amino acid sequence (SEQ ID NO: 1) encoded by the β-galactosidase gene of the plasmid pTG2ES-105, an amino acid sequence homology search was carried out by the use of NBRF-PIR of DNASIS™ (manufactured by Hitachi Software Engineering).

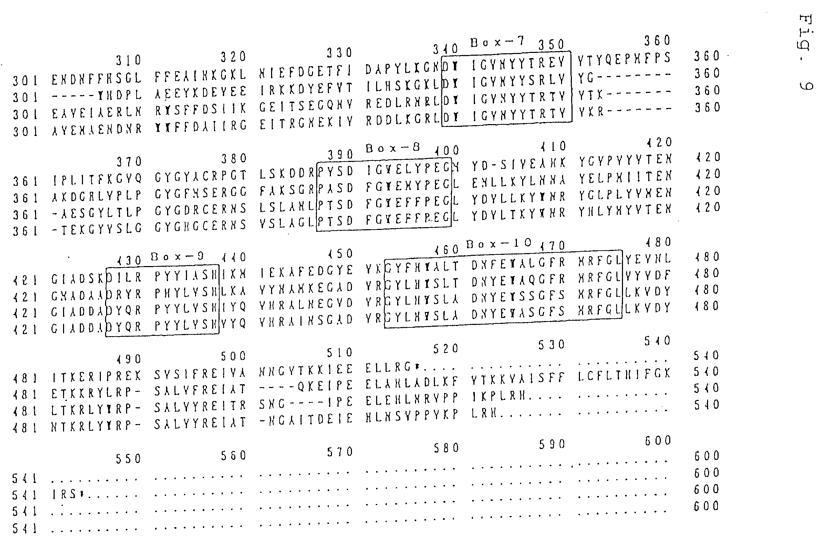

The amino acid sequences of the present enzyme and the other hyperthermostable β-galactosidase (SEQ ID NO: 3) produced by Pyrococcus furiosus were compared with these of two types of thermostable β-galactosidases (SEQ ID NO: 4 and SEQ ID NO: 5) present in Sulfolobus solfataricus, and it has for the first time become apparent that, surprisingly, some of the sequences homologous between two types of thermostable enzymes are preserved in the hyperthermostable enzyme. Figs. 8 and Fig. 9 are views comparing the amino acid sequences shown in SEQ ID NO: 1 and SEQ ID NO: 3 to SEQ ID NO: 5. The ten different sequences each designated a "box sequence" by the inventors as indicated in the Fig. 8 and Fig. 9 (Box No. 1 to Box No. 10) are the above preserved sequences. Other hyperthermostable β-galactosidase genes can be cloned on the basis of the above sequences, for example, by the use of a primer or probe prepared from the amino acid sequences of the Box Nos. 7, 8 and 10 respectively defined by the SEQ ID NO: 6, SEQ ID NO: 7 and SEQ ID NO: 8.

In Fig. 8 and Fig. 9, the four rows of amino acid sequences viewed from the top to the bottom respectively correspond to the SEQ ID NO: 3 (top row), the SEQ ID NO: 1 (second row), the SEQ ID NO: 4 (third row) and the SEQ ID NO: 5 (bottom row).

As described above in detail, the present invention provides a DNA or gene encoding a hyperthermostable β-galactosidase and a genetic engineering process for producing a hyperthermostable β-galactosidase by using said gene. This enzyme has a high thermostability and SDS-resistance and is useful particularly in food processing at high temperature and saccharide engineering.

Further, the DNA according to the present invention or a part thereof is also useful as a probe or primer for screening. Genes of all the enzymes analogous to the present enzyme which have sequences slightly different from that of the present enzyme but which are expected to have a similar enzymatic activity would be obtained by effecting hybridizations using the above obtained genes as the probe under strict conditions. The term "under strict conditions" as used herein means that the probe and hybridization of a nylon membrane having a DNA immobilized thereon are performed at 65 °C for 20 hr in a solution containing 6 x SSC (1 x SSC being a solution obtained by dissolving 8.76 g of sodium chloride and 4.41 g of sodium citrate in 1 l of water), 1% SDS, 100 mg/ml salmon sperm DNA and 5 x Denhardt's (containing each of bovine serum albumin, polyvinyl pyrrolidone and ficoll in a concentration of 0.1%).

Also, genes of all the enzymes analogous to the present enzyme which have sequences slightly different from that of the present enzyme but which are expected to have a similar enzymatic activity would be obtained by effecting gene amplification using the above obtained genes as the primer.

Moreover, screening can be performed with the use of an oligomer, as a probe, having a nucleotide sequence encoding the above amino acid sequence jointly preserved by the thermostable β-galactosidase and the hyperthermostable β-galactosidase. That is, any of the thermostable and hyperthermostable genes of the enzymes analogous to the present enzyme which are expected to have the same enzymatic activity as that of the present enzyme would be obtained from the thermophilic and hyperthermophilic bacteria, respectively, by carrying out hybridizations in the hybridization solution having the same composition as that mentioned above at a temperature 5 °C lower than the value of Tm at which each oligomer forms a complementary strand with the target DNA. Still further, screening can be performed by effecting gene amplification with the use of the above oligomer as a primer.

Whether the gene obtained by the above screening is the gene of an enzyme analogous to the present enzyme which is expected to have the same enzymatic activity as that of the present enzyme can be ascertained in the following manner. The obtained gene is ligated to an expression vector ensuring expression in a suitable host according to the conventional procedure and introduced into the host to thereby obtain a transformant. This transformant is cultured, and the β-galactosidase activity of the culture or a cell-free extract therefrom is measured by the method described herein. Thus, it can be ascertained whether this gene is the gene of an enzyme analogous to the present enzyme which is expected to have the same enzymatic activity as that of the present enzyme, i.e., which has a residual activity ratio of about 90% even after treatment at 90 °C for 120 minutes in the presence of SDS.

The hyperthermostable β-galactosidase obtained via the expression of the hyperthermostable β-galactosidase gene can be obtained by incubating a strain belonging to the genus Pyrococcus such as Pyrococcus furiosus DSM 3638 Pyrococcus woesei DSM 3773 in an appropriate growth medium and purifying the target enzyme from the cells or the culture broth. To incubate a bacterium of the genus Pyrococcus, a method usually employed for incubating a hyperthermostable bacterium may be used. Any nutrient which can be utilized by the employed strain may be added to the medium. For example, starch is usable as a carbon source and trypton and peptone are usable as a nitrogen source. As other nutrients, yeast extract and the like can be used. The medium may contain metal salts such as magnesium salts, sodium salts or iron salts as a trace element. It is advantageous to use artificial seawater for the preparation of the medium. The medium is preferably a transparent one free from a solid sulfur element, since such a medium makes it easy to monitor the growth of the cells by measuring the optical density of the culture. The incubation can be effected either stationarily or under stirring. For example, an aeration culture [WO 90/11352] or a dialysis culture [Applied and Environmental Microbiology, 55, 2086 - 2088 (1992)] may be carried out. In general, the incubation temperature is preferably around 95 °C. Usually, a considerably large amount of the hyperthermostable β-galactosidase is accumulated in the culture within about 16 hours. It is a matter of course that the incubation conditions should be determined in such a manner as to achieve the maximum yield of the hyperthermostable β-galactosidase depending on the selected strain and the composition of the medium.

The hyperthermostable β-galactosidase can be harvested by, for example, collecting the cells from the culture broth by centrifuging or filtering and then disrupting the cells. The cell disruption can be effected by, for example, ultrasonic disruption, bead disruption or lytic enzyme treatment. By using these techniques the hyperthermostable β-galactosidase can be extracted from the cells. The enzyme may be extracted by a method capable of giving the highest extraction effect depending on the selected bacterium and thus a crude enzyme solution is obtained. From the crude enzyme solution thus obtained, the hyperthermostable β-galactosidase can be isolated by combining techniques commonly employed for purifying enzymes, for example, salting out with ammonium sulfate, ion exchange chromatography, hydrophobic chromatography and gel filtration chromatography.

For example, a crude enzyme solution prepared from incubated cells of Pyrococcus furiosus DSM 3638 is chromatographed with a DEAE Toyopearl M650 ion exchanger (manufactured by Tosoh Corporation) to thereby elute an active fraction. The active fraction thus obtained is poured into an HIC-Cartridge Column (manufactured by Bio-Rad Laboratories) to thereby elute an active fraction. The active fraction thus eluted is poured into a Hydroxyapatite Column (manufactured by Bio-Rad Laboratories) to thereby elute an active fraction.

Thus the hyperthermostable β-galactosidase can be obtained.

### Brief Description of the Drawings

[Fig. 1]
   The figure showing a restriction enzyme cleavage map of the plasmid pTG2ES-105.
[Fig. 2]
   The figure showing a restriction enzyme cleavage map of one form of the hyperthermostable β-galactosidase gene.
[Fig. 3]
   The figure showing the thermostability of an enzyme.
[Fig. 4]
   The figure showing the optimum pH of an enzyme.
[Fig. 5]
   The figure showing the optimum temperature of an enzyme.
[Fig. 6]
   The figure showing the pH stability of an enzyme.
[Fig. 7]
   The figure showing the thermostability of an enzyme in the presence of a surfactant.
[Fig. 8]
   The figure showing part (first half) of a view comparing amino acid sequences of β-galactosidase.
[Fig. 9]
   The figure showing part (latter half) of a view comparing amino acid sequences of β-galactosidase.

### Examples

The following Example will further illustrate the present invention, which by no means limit the invention.

### Example 1 :

### (1) Preparation of Pyrococcus furiosus genome DNA

Pyrococcus furiosus DSM 3638 was incubated in the following manner.

2 l of a medium comprising 1% trypton, 0.5% yeast extract, 1% soluble starch, 3.5% Jamarin S Solid (manufactured by Jamarin Laboratory), 0.5% Jamarin S Liquid (manufactured by Jamarin Laboratory), 0.003% MgS0₄, 0.001% NaCl, 0.0001% FeS0₄ · 7H₂0, 0.0001% CoS0₄, 0.0001% CaCl₂ · 7H₂0, 0.0001% ZnS0₄, 0.1. ppm CuSO₄ · 5H₂0 , 0.1 ppm KAl(S0₄)₂, 0.1 ppm H₃B0₃, 0.1 ppm Na₂MoO₄ · 2H₂O and 0.25 ppm NiCl₂ · 6H₂O was fed into a 2 l medium bottle and sterilized at 120 °C for 20 minutes. After eliminating the dissolved oxygen by blowing nitrogen gas, the medium was inoculated with the above-mentioned strain, which was then stationarily incubated at 95 °C for 16 hours. After the completion of the incubation, cells were collected by centrifuging.

Then the collected cells were suspended in 4 ml of a 0.05 M Tris-HCl (pH 8.0) containing 25% sucrose. To the obtained suspension were added 0.8 ml of lysozyme [5 mg/ml, 0.25 M Tris-HCl (pH 8.0)] and 2 ml of 0.2 M EDTA. After maintaining at 20 °C for 1 hour, 24 ml of an SET solution [150 mM NaCl, 1 mM EDTA, 20 mM Tris-HCl (pH 8.0)] was added. Further, 4 ml of 5% SDS and 400 µl of proteinase K (10 mg/ml) were added thereto, followed by a reaction at 37 °C for 1 hour. After the completion of the reaction, the reaction mixture was extracted with chloroform/phenol and precipitated from ethanol. Thus approximately 3.2 mg of a genome DNA was prepared.

### (2) Preparation of cosmid protein library

400 µg of the Pyrococcus furiosus DSM 3638 genome DNA was partially digested with Sau 3AI in a buffer solution for Sau 3AI [50 mM Tris-HCl (pH 7.5), 10 mM MgCl₂, 1 mM dithiothreitol, 100 mM NaCl] and fractionated according to the size by density gradient centrifugation. 1 µg of Triple Helix Cosmid Vector was cleaved with Bam HI and mixed with 140 µg of the genome DNA fragments of 35 to 50 kbp which had been obtained by the fractionation as described above. After ligating with the use of a Ligation Kit (manufactured by Takara Shuzo Co., Ltd.), the Pyrococcus genome DNA fragments were packaged into λ-phage particles by the in vitro packaging method using Gigapack ®II Gold (manufactured by Stratagene). By using a part of the phage solution thus obtained,
Escherichia coli DH5αMCR was transformed to thereby give a cosmid library.

From several colonies thus obtained, cosmid DNAs were prepared and it was confirmed that they had an inserted fragment of an appropriate size in common. Next, 500 colonies were suspended in 2 ml of an L-broth medium containing 0.01% of ampicillin and incubated under shaking at 37 °C for 16 hours. The culture was centrifuged and cells were collected as a precipitate. These cells were suspended in 20 mM Tris-HCl (pH 8.0) and thermally treated at 100 °C for 10 minutes. Subsequently, they were ultrasonicated and further thermally treated at 100 °C for 10 minutes. After centrifuging, the supernatant was collected and referred to as a crude enzyme solution. Thus 500 cosmid protein libraries were prepared.

### (3) Selection of cosmid containing β-galactosidase gene

The β-galactosidase activity of the crude enzymatic solution of the 500 cosmid protein library obtained in Example 1-(2) was determined. Specifically, 10 µl of the crude enzymatic solution was added to 99.5 µl of a 100 mM phosphate buffer (pH 7.0) containing 112 mM 2-mercaptoethanol, 1 mM magnesium chloride and 1% SDS. Subsequently, 0.5 µl of a dimethyl sulfoxide solution containing 0.4 M o-nitrophenyl-β-D-galactopyranoside was added and reacted at 95 °C for 30 minutes. This reaction was terminated by adding 50 µl of 0.1 M sodium carbonate. The absorbance at 410 nm was measured, thereby determining the amount of the formed o-nitrophenol.

One cosmid Protein with β-galactosidase activity was selected from the 500 cosmid protein library and one cosmid DNA corresponding thereto was identified.

### (4) Preparation of plasmid PTG2S-112 and production of thermostable β-galactosidase

The one cosmid DNA obtained in Example 1-(3) was completely digested with the restriction enzyme Sma I. Separately, pUC18 as a vector was cleaved at its Sma I site, followed by end dephosphorylation. The above Sma I digested DNA fragment was ligated to the vector plasmid by the use of a ligation kit. The Escherichia coli JM109 was transformed with the use of the resultant reaction solution. The transformant was suspended in 5 ml of an L-broth medium containing 0.01% ampicillin and cultured while shaking at 37 °C for 16 hr. The resultant culture was centrifuged, and the recovered cells were suspended in a 50 mM phosphate buffer (pH 7.0) containing 1 mM EDTA. The suspension was heated at 100 °C for 10 minutes, sonicated, further heated at 100 °C for 10 minutes, and centrifuged to thereby obtain a supernatant as a crude enzymatic solution. The β-galactosidase activity was assayed by the same activity assay method as that of Example 1-(3) except that 5 µl of this crude enzymatic solution was used. The hyperthermostable β-galactosidase activity exhibiting resistance to heat treatment at 100 °C for 20 minutes was recognized in the crude enzymatic solution.

The plasmid corresponding to this crude enzymatic solution was designated plasmid pTG2S-112. The plasmid pTG2S-112 was introduced into the Escherichia coli JM109, thereby obtaining a transformant. This transformant was designated as Escherichia coli JM109/pTG2S-112.

### (5) Preparation of plasmid pTG2ES-105

The plasmid pTG2S-112 containing the Sma I DNA fragment of about 4 kbp obtained in Example 1-(4) was completely digested with the restriction enzymes Eco81I and Kpn I. The resultant Eco81 I-Kpn I DNA fragment of about 4.7 kbp was purified, blunt-ended and self-ligated.

The obtained plasmid was designated plasmid pTG2ES-105. This plasmid was introduced into the Escherichia coli JM109, thereby obtaining a transformant. This transformant was designated as Escherichia coli JM109/pTG2ES-105. This strain was deposited on April 20, 1994 at National Institute of Bioscience and Human-Technology Agency of Industrial Science and Technology (1-3, Higashi 1 Chome Tsukuba-shi Ibaraki-ken 305, JAPAN) under the accession number FERM BP-5023.

Fig. 1 shows a restriction enzyme cleavage map of the plasmid pTG2ES-105, and Fig. 2 shows a restriction enzyme cleavage map of the hyperthermostable β-galactosidase gene of about 2.0 kbp obtained according to the present invention which was derived from Pyrococcus furiosus and inserted in the plasmid pTG2ES-105.

### Example 2 :

### (Determination of nucleotide sequence of hyperthermostable β-galactosidase gene)

Deletion mutants were prepared from the above fragment of about 2.0 kbp including the hyperthermostable β-galactosidase gene inserted in the plasmid pTG2ES-105 with the use of Deletion Kit for Kilo Sequence (manufactured by Takara Shuzo Co., Ltd.), and the nucleotide sequences of the resultant fragments were determined.

The determination of the nucleotide sequences was conducted by the dideoxy method in which use was made of the Bca Bast™ Dideoxy Sequencing Kit (manufactured by Takara Shuzo Co., Ltd.).

SEQ ID NO: 2 shows in the nucleotide sequence of the DNA fragment including the hyperthermostable β-galactosidase gene inserted in the plasmid pTG2ES-105. SEQ ID NO: 1 shows in the amino acid sequence of the hyperthermostable β-galactosidase encoded for by the above nucleotide sequence.

### Example 3 :

### (1) Production of hyperthermostable β-galactosidase

The Escherichia coli JM109/pTG2ES-105 (FERM BP-5023) obtained in Example 1, into which the plasmid pTG2ES-105 containing the hyperthermostable β-galactosidase gene of the present invention had been introduced, was suspended in 5 ml of an L-broth medium containing 0.01% ampicillin and cultured while shaking at 37 °C for 16 hr. The culture was suspended in 1.2 1 of the medium of the same composition and cultured while shaking at 37 °C for 16 hr. The resultant culture was centrifuged, and the recovered cells were suspended in a 50 mM phosphate buffer (pH 7.0) containing 1 mM EDTA. The suspension was heated at 100 °C for 10 minutes, sonicated, further heated at 100 °C for 10 minutes, and centrifuged to thereby obtain a supernatant as a crude enzymatic solution.

The specific activity of β-galactosidase in the crude enzymatic solution was about 740 units/mg at pH 5.0 and 90 °C.

### Effect of the Invention

An SDS-resistant hyperthermostable β-galactosidase can advantageously be produced on a commercial scale by the use of the hyperthermostable β-galactosidase gene.

Moreover, various biologically derived hyperthermostable β-galactosidase genes can be obtained by the use of the hyperthermostable β-galactosidase gene or a part thereof as a probe or primer.

### SEQUENCE LISTING

SEQ IN NO : 1
LENGTH : 491
TYPE : amino acid
STRANDEDNESS : single
TOPOLOGY : linear
MOLECULE TYPE : peptide
SEQUENCE DESCRIPTION : SEQ ID NO : 1 
Met Lys Phe Pro Lys Asn Phe Met Phe Gly Tyr Ser Trp Ser Gly
1               5                   10                  15
Phe Gln Phe Glu Met Gly Leu Pro Gly Ser Glu Val Glu Ser Asp
                20                  25                  30
Trp Trp Val Trp Val His Asp Lys Glu Asn Ile Ala Ser Gly Leu
                35                  40                  45
Val Ser Gly Asp Leu Pro Glu Asn Gly Pro Ala Tyr Trp His Leu
                50                  55                  60
Tyr Lys Gln Asp His Asp Ile Ala Glu Lys Leu Gly Met Asp Cys
                65                  70                  75
Ile Arg Gly Gly Ile Glu Trp Ala Arg Ile Phe Pro Lys Pro Thr
                80                  85                  90
Phe Asp Val Lys Val Asp Val Glu Lys Asp Glu Glu Gly Asn Ile
                95                  100                 105
Ile Ser Val Asp Val Pro Glu Ser Thr Ile Lys Glu Leu Glu Lys
                110                 115                 120
Ile Ala Asn Met Glu Ala Leu Glu His Tyr Arg Lys Ile Tyr Ser
                125                 130                 135
Asp Trp Lys Glu Arg Gly Lys Thr Phe Ile Leu Asn Leu Tyr His
                140                 145                 150
 
Trp Pro Leu Pro Leu Trp Ile His Asp Pro Ile Ala Val Arg Lys
                155                 160                 165
Leu Gly Pro Asp Arg Ala Pro Ala Gly Trp Leu Asp Glu Lys Thr
                170                 175                 180
Val Val Glu Phe Val Lys Phe Ala Ala Phe Val Ala Tyr His Leu
                185                 190                 195
Asp Asp Leu Val Asp Met Trp Ser Thr Met Asn Glu Pro Asn Val
                200                 205                 210
Val Tyr Asn Gln Gly Tyr Ile Asn Leu Arg Ser Gly Phe Pro Pro
                215                 220                 225
Gly Tyr Leu Ser Phe Glu Ala Ala Glu Lys Ala Lys Phe Asn Leu
                230                 235                 240
Ile Gln Ala His Ile Gly Ala Tyr Asp Ala Ile Lys Glu Tyr Ser
                245                 250                 255
Glu Lys Ser Val Gly Val Ile Tyr Ala Phe Ala Trp His Asp Pro
                260                 265                 270
Leu Ala Glu Glu Tyr Lys Asp Glu Val Glu Glu Ile Arg Lys Lys
                275                 280                 285
Asp Tyr Glu Phe Val Thr Ile Leu His Ser Lys Gly Lys Leu Asp
                290                 295                 300
Trp Ile Gly Val Asn Tyr Tyr Ser Arg Leu Val Tyr Gly Ala Lys
                305                 310                 315
Asp Gly His Leu Val Pro Leu Pro Gly Tyr Gly Phe Met Ser Glu
                320                 325                 330
Arg Gly Gly Phe Ala Lys Ser Gly Arg Pro Ala Ser Asp Phe Gly
                335                 340                 345
Trp Glu Met Tyr Pro Glu Gly Leu Glu Asn Leu Leu Lys Tyr Leu
                350                 355                 360
Asn Asn Ala Tyr Glu Leu Pro Met Ile Ile Thr Glu Asn Gly Met
 
                365                 370                 375
Ala Asp Ala Ala Asp Arg Tyr Arg Pro His Tyr Leu Val Ser His
                380                 385                 390
Leu Lys Ala Val Tyr Asn Ala Met Lys Glu Gly Ala Asp Val Arg
                395                 400                 405
Gly Tyr Leu His Trp Ser Leu Thr Asp Asn Tyr Glu Trp Ala Gln
                410                 415                 420
Gly Phe Arg Met Arg Phe Gly Leu Val Tyr Val Asp Phe Glu Thr
                425                 430                 435
Lys Lys Arg Tyr Leu Arg Pro Ser Ala Leu Val Phe Arg Glu Ile
                440                 445                 450
Ala Thr Gln Lys Glu Ile Pro Glu Glu Leu Ala His Leu Ala Asp
                455                 460                 465
Leu Lys Phe Val Thr Lys Lys Val Ala Ile Ser Phe Phe Leu Cys
                470                 475                 480
Phe Leu Thr His Ile Phe Gly Lys Ile Arg Ser
                485                 490
 SEQ ID NO : 2
LENGTH : 1476
TYPE : nucleic acid
STRANDEDNESS : double
TOPOLOGY : linear
MOLECULE TYPE : Genomic DNA
SEQUENCE DESCRIPTION : SEQ ID NO : 2 
ATGAAGTTCC CAAAAAACTT CATGTTTGGA TATTCTTGGT CTGGTTTCCA GTTTGAGATG 60
GGACTGCCAG GAAGTGAAGT GGAAAGCGAC TGGTGGGTGT GGGTTCACGA CAAGGAGAAC 120
ATAGCATCAG GTCTAGTAAG TGGAGATCTA CCAGAGAACG GCCCAGCATA TTGGCACCTC 180
TATAAGCAAG ATCATGACAT TGCAGAAAAG CTAGGAATGG ATTGTATTAG AGGTGGCATT 240
 
GAGTGGGCAA GAATTTTTCC AAAGCCAACA TTTGACGTTA AAGTTGATGT GGAAAAGGAT 300
GAAGAAGGCA ACATAATTTC CGTAGACGTT CCAGAGAGTA CAATAAAAGA GCTAGAGAAA 360
ATTGCCAACA TGGAGGCCCT TGAACATTAT CGCAAGATTT ACTCAGACTG GAAGGAGAGG 420
GGCAAAACCT TCATATTAAA CCTCTACCAC TGGCCTCTTC CATTATGGAT TCATGACCCA 480
ATTGCAGTAA GGAAACTTGG CCCGGATAGG GCTCCTGCAG GATGGTTAGA TGAGAAGACA 540
GTGGTAGAGT TTGTGAAGTT TGCCGCCTTC GTTGCTTATC ACCTTGATGA CCTCGTTGAC 600
ATGTGGAGCA CAATGAACGA ACCAAACGTA GTCTACAATC AAGGTTACAT TAATCTACGT 660
TCAGGATTTC CACCAGGATA TCTAAGCTTT GAAGCAGCAG AAAAGGCAAA ATTCAACTTA 720
ATTCAGGCTC ACATCGGAGC ATATGATGCC ATAAAAGAGT ATTCAGAAAA ATCCGTGGGA 780
GTGATATACG CCTTTGCTTG GCACGATCCT CTAGCGGAGG AGTATAAGGA TGAAGTAGAG 840
GAAATCAGAA AGAAAGACTA TGAGTTTGTA ACAATTCTAC ACTCAAAAGG AAAGCTAGAC 900
TGGATCGGCG TAAACTACTA CTCCAGGCTG GTATATGGAG CCAAAGATGG ACACCTAGTT 960
CCTTTACCTG GATATGGATT TATGAGTGAG AGAGGAGGAT TTGCAAAGTC AGGAAGACCT 1020
GCTAGTGACT TTGGATGGGA AATGTACCCA GAGGGCCTTG AGAACCTTCT TAAGTATTTA 1080
AACAATGCCT ACGAGCTACC AATGATAATT ACAGAGAACG GTATGGCCGA TGCAGCAGAT 1140
AGATACAGGC CACACTATCT CGTAAGCCAT CTAAAGGCAG TTTACAATGC TATGAAAGAA 1200
GGTGCTGATG TTAGAGGGTA TCTCCACTGG TCTCTAACAG ACAACTACGA ATGGGCCCAA 1260
GGGTTCAGGA TGAGATTTGG ATTGGTTTAC GTGGATTTCG AGACAAAGAA GAGATATTTA 1320
AGGCCAAGCG CCCTGGTATT CAGAGAAATA GCCACTCAAA AAGAAATTCC AGAAGAATTA 1380
GCTCACCTCG CAGACCTCAA ATTTGTTACC AAGAAAGTAG CCATTTCATT TTTTCTTTGT 1440
TTTTTAACTC ATATTTTTGG GAAAATAAGA TCATAA 1476
 SEQ ID NO : 3
LENGTH : 510
TYPE : amino acid
STRANDEDNESS : single
TOPOLOGY : linear
MOLECULE TYPE : Peptide
SEQUENCE DESCRIPTION : SEQ ID NO : 3 
Met Phe Pro Glu Lys Phe Leu Trp Gly Val Ala Gln Ser Gly Phe
1               5                   10                  15
Gln Phe Glu Met Gly Asp Lys Leu Arg Arg Asn Ile Asp Thr Asn
                20                  25                  30
Thr Asp Trp Trp His Trp Val Arg Asp Lys Thr Asn Ile Glu Lys
                35                  40                  45
Gly Leu Val Ser Gly Asp Leu Pro Glu Glu Gly Ile Asn Asn Tyr
                50                  55                  60
Glu Leu Tyr Glu Lys Asp His Glu Ile Ala Arg Lys Leu Gly Leu
                65                  70                  75
Asn Ala Tyr Arg Ile Gly Ile Glu Trp Ser Arg Ile Phe Pro Trp
                80                  85                  90
Pro Thr Thr Phe Ile Asp Val Asp Tyr Ser Tyr Asn Glu Ser Tyr
                95                  100                 105
Asn Leu Ile Glu Asp Val Lys Ile Thr Lys Asp Thr Leu Glu Glu
                110                 115                 120
Leu Asp Glu Ile Ala Asn Lys Arg Glu Val Ala Tyr Tyr Arg Ser
                125                 130                 135
Val Ile Asn Ser Leu Arg Ser Lys Gly Phe Lys Val Ile Val Asn
                140                 145                 150
Leu Asn His Phe Thr Leu Pro Tyr Trp Leu His Asp Pro Ile Glu
                155                 160                 165
Ala Arg Glu Arg Ala Leu Thr Asn Lys Arg Asn Gly Trp Val Asn
                170                 175                 180
Pro Arg Thr Val Ile Glu Phe Ala Lys Tyr Ala Ala Tyr Ile Ala
                185                 190                 195
Tyr Lys Phe Gly Asp Ile Val Asp Met Trp Ser Thr Phe Asn Glu
                200                 205                 210
Pro Met Val Val Val Glu Leu Gly Tyr Leu Ala Pro Tyr Ser Gly
 
                215                 220                 225
Phe Pro Pro Gly Val Leu Asn Pro Glu Ala Ala Lys Leu Ala Ile
                230                 235                 240
Leu His Met Ile Asn Ala His Ala Leu Ala Tyr Arg Gln Ile Lys
                245                 250                 255
Lys Phe Asp Thr Glu Lys Ala Asp Lys Asp Ser Lys Glu Pro Ala
                260                 265                 270
Glu Val Gly Ile Ile Tyr Asn Asn Ile Gly Val Ala Tyr Pro Lys
                275                 280                 285
Asp Pro Asn Asp Ser Lys Asp Val Lys Ala Ala Glu Asn Asp Asn
                290                 295                 300
Phe Phe His Ser Gly Leu Phe Phe Glu Ala Ile His Lys Gly Lys
                305                 310                 315
Leu Asn Ile Glu Phe Asp Gly Glu Thr Phe Ile Asp Ala Pro Tyr
                320                 325                 330
Leu Lys Gly Asn Asp Trp Ile Gly Val Asn Tyr Tyr Thr Arg Glu
                335                 340                 345
Val Val Thr Tyr Gln Glu Pro Met Phe Pro Ser Ile Pro Leu Ile
                350                 355                 360
Thr Phe Lys Gly Val Gln Gly Tyr Gly Tyr Ala Cys Arg Pro Gly
                365                 370                 375
Thr Leu Ser Lys Asp Asp Arg Pro Val Ser Asp Ile Gly Trp Glu
                380                 385                 390
Leu Tyr Pro Glu Gly Met Tyr Asp Ser Ile Val Glu Ala His Lys
                395                 400                 405
Tyr Gly Val Pro Val Tyr Val Thr Glu Asn Gly Ile Ala Asp Ser
                410                 415                 420
Lys Asp Ile Leu Arg Pro Tyr Tyr Ile Ala Ser His Ile Lys Met
                425                 430                 435
 
Ile Glu Lys Ala Phe Glu Asp Gly Tyr Glu Val Lys Gly Tyr Phe
                440                 445                 450
His Trp Ala Leu Thr Asp Asn Phe Glu Trp Ala Leu Gly Phe Arg
                455                 460                 465
Met Arg Phe Gly Leu Tyr Glu Val Asn Leu Ile Thr Lys Glu Arg
                470                 475                 480
Ile Pro Arg Glu Lys Ser Val Ser Ile Phe Arg Glu Ile Val Ala
                485                 490                 495
Asn Asn Gly Val Thr Lys Lys Ile Glu Glu Glu Leu Leu Arg Gly
                500                 505                 510
 SEQ ID NO : 4
LENGTH : 491
TYPE : amino acid
STRANDEDNESS : single
TOPOLOGY : linear
MOLECULE TYPE : peptide
SEQUENCE DESCRIPTION : SEQ ID NO : 4 
Met Leu Ser Phe Pro Lys Gly Phe Lys Phe Gly Trp Ser Gln Ser
1               5                   10                  15
Gly Phe Gln Ser Glu Met Gly Thr Pro Gly Ser Glu Asp Pro Asn
                20                  25                  30
Ser Asp Trp His Val Trp Val His Asp Arg Glu Asn Ile Val Ser
                35                  40                  45
Gln Val Val Ser Gly Asp Leu Pro Glu Asn Gly Pro Gly Tyr Trp
                50                  55                  60
Gly Asn Tyr Lys Arg Phe His Asp Glu Ala Glu Lys Ile Gly Leu
                65                  70                  75
Asn Ala Val Arg Ile Asn Val Glu Trp Ser Arg Ile Phe Pro Arg
 
                80                  85                  90
Pro Leu Pro Lys Pro Glu Met Gln Thr Gly Thr Asp Lys Glu Asn
                95                  100                 105
Ser Pro Val Ile Ser Val Asp Leu Asn Glu Ser Lys Leu Arg Glu
                110                 115                 120
Met Asp Asn Tyr Ala Asn His Glu Ala Leu Ser His Tyr Arg His
                125                 130                 135
Ile Leu Glu Asp Leu Arg Asn Arg Gly Phe His Ile Val Leu Asn
                140                 145                 150
Met Tyr His Trp Thr Leu Pro Ile Trp Leu His Asp Pro Ile Arg
                155                 160                 165
Val Arg Arg Gly Asp Phe Thr Gly Pro Thr Gly Trp Leu Asn Ser
                170                 175                 180
Arg Thr Val Tyr Glu Phe Ala Arg Phe Ser Ala Tyr Val Ala Trp
                185                 190                 195
Lys Leu Asp Asp Leu Ala Ser Glu Tyr Ala Thr Met Asn Glu Pro
                200                 205                 210
Asn Val Val Trp Gly Ala Gly Tyr Ala Phe Pro Arg Ala Gly Phe
                215                 220                 225
Pro Pro Asn Tyr Leu Ser Phe Arg Leu Ser Glu Ile Ala Lys Trp
                230                 235                 240
Asn Ile Ile Gln Ala His Ala Arg Ala Tyr Asp Ala Ile Lys Ser
                245                 250                 255
Val Ser Lys Lys Ser Val Gly Ile Ile Tyr Ala Asn Thr Ser Tyr
                260                 265                 270
Tyr Pro Leu Arg Pro Gln Asp Asn Glu Ala Val Glu Ile Ala Glu
                275                 280                 285
Arg Leu Asn Arg Trp Ser Phe Phe Asp Ser Ile Ile Lys Gly Glu
                290                 295                 300
 
Ile Thr Ser Glu Gly Gln Asn Val Arg Glu Asp Leu Arg Asn Arg
                305                 310                 315
Leu Asp Trp Ile Gly Val Asn Tyr Tyr Thr Arg Thr Val Val Thr
                320                 325                 330
Lys Ala Glu Ser Gly Tyr Leu Thr Leu Pro Gly Tyr Gly Asp Arg
                335                 340                 345
Cys Glu Arg Asn Ser Leu Ser Leu Ala Asn Leu Pro Thr Ser Asp
                350                 355                 360
Phe Gly Trp Glu Phe Phe Pro Glu Gly Leu Tyr Asp Val Leu Leu
                365                 370                 375
Lys Tyr Trp Asn Arg Tyr Gly Leu Pro Leu Tyr Val Met Glu Asn
                380                 385                 390
Gly Ile Ala Asp Asp Ala Asp Tyr Gln Arg Pro Tyr Tyr Leu Val
                395                 400                 405
Ser His Ile Tyr Gln Val His Arg Ala Leu Asn Glu Gly Val Asp
                410                 415                 420
Val Arg Gly Tyr Leu His Trp Ser Leu Ala Asp Asn Tyr Glu Trp
                425                 430                 435
Ser Ser Gly Phe Ser Met Arg Phe Gly Leu Leu Lys Val Asp Tyr
                440                 445                 450
Leu Thr Lys Arg Leu Tyr Trp Arg Pro Ser Ala Leu Val Tyr Arg
                455                 460                 465
Glu Ile Thr Arg Ser Asn Gly Ile Pro Glu Glu Leu Glu His Leu
                470                 475                 480
Asn Arg Val Pro Pro Ile Lys Pro Leu Arg His
                485                 490
 SEQ ID NO : 5
LENGTH : 489
TYPE : amino acid
STRANDEDNESS : single
TOPOLOGY : linear
MOLECULE TYPE : peptide
SEQUENCE DESCRIPTION : SEQ ID NO : 5 
Met Tyr Ser Phe Pro Asn Ser Phe Arg Phe Gly Trp Ser Gln Ala
1               5                   10                  15
Gly Phe Gln Ser Glu Met Gly Thr Pro Gly Ser Glu Asp Pro Asn
                20                  25                  30
Thr Asp Trp Tyr Lys Trp Val His Asp Pro Glu Asn Met Ala Ala
                35                  40                  45
Gly Leu Val Ser Gly Asp Leu Pro Glu Asn Gly Pro Gly Tyr Trp
                50                  55                  60
Gly Asn Tyr Lys Thr Phe His Asp Asn Ala Gln Lys Met Gly Leu
                65                  70                  75
Lys Ile Ala Arg Leu Asn Val Glu Trp Ser Arg Ile Phe Pro Asn
                80                  85                  90
Pro Leu Pro Arg Pro Gln Asn Phe Asp Glu Ser Lys Gln Asp Val
                95                  100                 105
Thr Glu Val Glu Ile Asn Glu Asn Glu Leu Lys Arg Leu Asp Glu
                110                 115                 120
Tyr Ala Asn Lys Asp Ala Leu Asn His Tyr Arg Glu Ile Phe Lys
                125                 130                 135
Asp Leu Lys Ser Arg Gly Leu Tyr Phe Ile Leu Asn Met Tyr His
                140                 145                 150
Trp Pro Leu Pro Leu Trp Leu His Asp Pro Ile Arg Val Arg Arg
                155                 160                 165
Gly Asp Phe Thr Gly Pro Ser Gly Trp Leu Ser Thr Arg Thr Val
                170                 175                 180
 
Tyr Glu Phe Ala Arg Phe Ser Ala Tyr Ile Ala Trp Lys Phe Asp
                185                 190                 195
Asp Leu Val Asp Glu Tyr Ser Thr Met Asn Glu Pro Asn Val Val
                200                 205                 210
Gly Gly Leu Gly Tyr Val Gly Val Lys Ser Gly Phe Pro Pro Gly
                215                 220                 225
Tyr Leu Ser Phe Glu Leu Ser Arg Arg His Met Tyr Asn Ile Ile
                230                 235                 240
Gln Ala His Ala Arg Ala Tyr Asp Gly Ile Lys Ser Val Ser Lys
                245                 250                 255
Lys Pro Val Gly Ile Ile Tyr Ala Asn Ser Ser Phe Gln Pro Leu
                260                 265                 270
Thr Asp Lys Asp Met Glu Ala Val Glu Met Ala Glu Asn Asp Asn
                275                 280                 285
Arg Trp Trp Phe Phe Asp Ala Ile Ile Arg Gly Glu Ile Thr Arg
                290                 295                 300
Gly Asn Glu Lys Ile Val Arg Asp Asp Leu Lys Gly Arg Leu Asp
                305                 310                 315
Trp Ile Gly Val Asn Tyr Tyr Thr Arg Thr Val Val Lys Arg Thr
                320                 325                 330
Glu Lys Gly Tyr Val Ser Leu Gly Gly Tyr Gly His Gly Cys Glu
                335                 340                 345
Arg Asn Ser Val Ser Leu Ala Gly Leu Pro Thr Ser Asp Phe Gly
                350                 355                 360
Trp Glu Phe Phe Pro Glu Gly Leu Tyr Asp Val Leu Thr Lys Tyr
                365                 370                 375
Trp Asn Arg Tyr His Leu Tyr Met Tyr Val Thr Glu Asn Gly Ile
                380                 385                 390
Ala Asp Asp Ala Asp Tyr Gln Arg Pro Tyr Tyr Leu Val Ser His
 
                395                 400                 405
Val Tyr Gln Val His Arg Ala Ile Asn Ser Gly Ala Asp Val Arg
                410                 415                 420
Gly Tyr Leu His Trp Ser Leu Ala Asp Asn Tyr Glu Trp Ala Ser
                425                 430                 435
Gly Phe Ser Met Arg Phe Gly Leu Leu Lys Val Asp Tyr Asn Thr
                440                 445                 450
Lys Arg Leu Tyr Trp Arg Pro Ser Ala Leu Val Tyr Arg Glu Ile
                455                 460                 465
Ala Thr Asn Gly Ala Ile Thr Asp Glu Ile Glu His Leu Asn Ser
                470                 475                 480
Val Pro Pro Val Lys Pro Leu Arg His
                485
 SEQ ID NO : 6
LENGTH : 12
TYPE : amino acid
STRANDEDNESS : single
TOPOLOGY : linear
MOLECUEL TYPE : peptide
SEQUENCE DESCRIPTION : SEQ ID NO : 6 
Asp Trp Ile Gly Val Asn Tyr Tyr Ser Arg Leu Val
1               5                   10
 SEQ ID NO : 7
LENGTH : 13
TYPE : amino acid
STRANDEDNESS : single
TOPOLOGY : linear
MOLECULE TYPE : peptide
SEQUENCE DESCRIPTION : SEQ ID NO : 7 
Pro Ala Ser Asp Phe Gly Trp Glu Met Tyr Pro Glu Gly
1               5                   10
 SEQ ID NO : 8
LENGTH : 23
TYPE : amino acid
STRANDEDNESS : single
TOPOLOGY : linear
MOLECULE TYPE : peptide
SEQUENCE DESCRIPTION : SEQ ID NO : 8

Gly Tyr Leu His Trp Ser Leu·Thr Asp Asn Tyr Glu Trp Ala Gln
1 5 10 15
Gly Phe Arg Met Arg Phe Gly Leu
20

## Claims

1. A DNA having:
(a) the DNA sequence of SEQ ID NO 2, or a fragment thereof;
(b) a DNA Sequence encoding the amino acid sequence of SEQ ID NO 1 or a fragment thereof;
(c) a DNA sequence encoding an amino acid sequence resulting from deletion, addition, insertion or substitution of one or more amino acids in the amino acid sequence of SEQ ID NO 1; or
(d) a DNA sequence capable of hybridizing of any one of (a) to (c)
wherein said DNA has a sequence encoding a polypeptide possessing SDS-resistant hyperthermostable β-galactosidase activity of about 90% after having been treated in the presence of 1% SDS at 90°C for 120 minutes.

2. A method of cloning a DNA having a sequence encoding a polypeptide possessing SDS-resistant hyperthermostable β-galactosidase activity in the presence of 1% SDS at 90°C for 120 minutes, which comprises using a DNA as claimed in Claim 1 or a part thereof as a probe or primer.

3. A process for producing a polypeptide possessing SDS-resistant hyperthermostable β-galactosidase activity in the presence of 1% SDS at 90°C for 120 minutes, which comprises culturing a transformant, into which a recombinant plasmid containing a DNA as claimed in claim 1 has been introduced, and harvesting an SDS-resistant hyperthermostable β-galactosidase from the culture.

## Patentansprüche

1. DNA mit
(a) der DNA-Sequenz SEQ ID Nr. 2 oder einem Fragment davon;
(b) einer die Aminosäuresequenz SEQ ID Nr. 1 oder ein Fragment davon kodierenden DNA-Sequenz;
(c) einer DNA-Sequenz, welche eine Aminosäuresequenz, resultierend aus Deletieren, Addieren, Insertieren oder Substitutieren einer oder mehrerer Aminosäuren in der Aminosäuresequenz SEQ ID Nr. 1, kodiert; oder
(d) einer DNA-Sequenz, befähigt zum Hybridisieren mit jeder von (a) bis (c),
worin diese DNA eine Sequenz aufweist, welche ein Polypeptid kodiert, welches SDS-resistente, hyperthermostabile β-Galaktosidaseaktivität von etwa 90% nach 120 min Behandlung in Gegenwart von 1% SDS bei 90°C besitzt.

2. Verfahren zum Klonieren einer DNA mit einer Sequenz, welche ein Polypeptid kodiert, welches SDS-resistente hyperthermostabile β-Galaktosidaseaktivität in Gegenwart von 1% SDS bei 90°C für 120 min besitzt, umfassend das Verwenden einer DNA gemäß Anspruch 1 oder eines Teils davon als Sonde oder Primer.

3. Verfahren zur Herstellung eines Polypeptids, welches SDS-resistente, hyperthermostabile β-Galaktosidaseaktivität in Gegenwart von 1 % SDS bei 90°C für 120 min besitzt, welches das Züchten eines Transformanten, in welchen ein eine DNA gemäß Anspruch 1 enthaltendes rekombinantes Plasmid eingebracht wurde, und das Ernten einer SDS-resistenten, hyperthermostabilen β-Galaktosidase von dieser Kultur umfaßt.

## Revendications

1. ADN ayant
(a) la séquence d'ADN correspondant à la Séquence identifiée par le N° 2, ou un fragment de celle-ci ;
(b) une séquence d'ADN codant pour la séquence d'acides aminés correspondant à la Séquence identifiée par le N° 1 ou un fragment de celle-ci ;
(c) une séquence d'ADN codant pour une séquence d'acides aminés résultant d'une délétion, addition, insertion ou substitution d'un ou de plusieurs acides aminés dans la séquence d'acides aminés correspondant à la Séquence identifiée par le N° 1 ; ou
(d) une séquence d'ADN capable de s'hybrider à l'une quelconque des séquences (a) à (c),
dans lequel ledit ADN a une séquence codant pour un polypeptide possédant une activité de type β-galactosidase hyperthermostable résistante au SDS d'environ 90% à la suite de traitement à 90°C pendant 120 minutes en présence de 1% de SDS.

2. Procédé de clonage d'un ADN ayant une séquence codant pour un polypeptide possédant une activité de type β-galactosidase hyperthermostable résistante au SDS en présence de 1% de SDS à 90°C pendant 120 minutes, qui comprend l'utilisation d'un ADN selon la revendication 1 ou une partie de celui-ci à titre de sonde ou d'amorce.

3. Procédé de production d'un polypeptide possédant une activité de type β-galactosidase hyperthermostable résistante au SDS en présence de 1% de SDS à 90°C pendant 120 minutes, qui comprend la mise en culture d'un transformant, dans lequel un plasmide recombinant contenant un ADN selon la revendication 1 a été introduit, et l'isolement d'une β-galactosidase hyperthermostable résistante au SDS à partir de la culture.
